# EUROPEAN PATENT APPLICATION

(11) **EP 1 527 732 A1**
(43) Date of publication of application: **04.05.2005**
(21) Application number: 03784595.5
(22) Date of filing: 08.08.2003
(51) Int. Cl.: A61B 5/00, A61B 5/145, G06F 17/60

(54) **DATA MEASURING DEVICE, HEALTHCARE DATA ACQUIRING SYSTEM, AND HEALTHCARE DATA ACQUIRING METHOD**

(30) Priority: 09.08.2002 JP 2002233369
(71) Applicant: MATSUSHITA ELECTRIC INDUSTRIAL CO., LTD., Kadoma-shi, Osaka 571-8501 (JP)
(72) Inventor: TSUTSUI, Hiroshi, Yawata-shi, Kyoto 614-8327 (JP); MORI, Yasuhiro, Izumi-shi, Osaka 594-0073 (JP); YAMAMOTO, Hirosi, Shijonawate-shi, Osaka 575-0013 (JP); NANKAI, Shiro, Hirakata-shi, Osaka 573-0071 (JP); SHIGETOH, Nobuyuki, Kyotanabe-shi, Kyoto 610-0354 (JP); KAWAMURA, Tatsurou, Kyotanabe-shi, Kyoto 610-0351 (JP)
(74) Representative: Balsters, Robert
(86) International application number: PCT/JP2003/010110
(87) International publication number: WO 2004/014228

(57) **Abstract**

The data measuring unit 11 measures a subject's blood and urine, and acquires numerical data (S501). The communication control unit 12 transmits the acquired numerical data to the data management apparatus 20 (S502). The overall control unit 14 receives the numerical data from the data measuring apparatus 10 (S503), and analyzes the content of the received numerical data (S504). The overall control unit 14 selects calibration curve data based on the above mentioned analysis result (S505), and calculates health care data (S506). Furthermore, the overall control unit 14 directs the communication control unit 13 to transmit the calculated health care data to the data measuring apparatus 10 (S507). The data measuring apparatus 10 receives the health care data from the data management apparatus 20 (S508).

## Description

### Technical Field

The present invention relates to a data measuring apparatus which is capable of an easy health check without being restricted by a place or time and to a health care data acquisition system and the like.

### Background Art

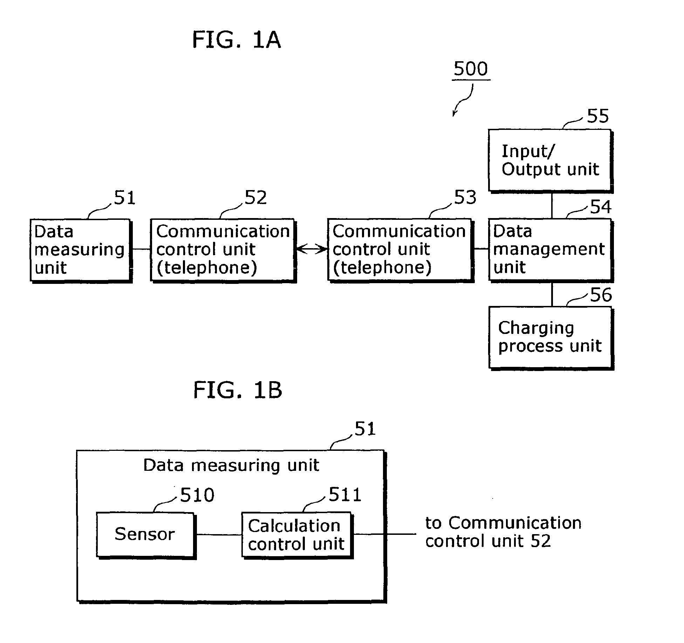

Conventionally, a system and the like have been suggested and actually operating, said system comprising the following steps. First, data concerning health such as blood pressure, pulse rate, blood glucose level and the like is measured by a measuring apparatus. Then, the data is transmitted to a medical institution or an autonomous community via a telephone line and the like. And, medical information such as a diagnosis, advice and the like is provided at the medical institution and the like. A representative functional structure of the conventional medical information service system 500 is shown in FIG. 1(a).

As shown in FIG. 1(a), the medical information service system 500 comprises the data measuring unit 51, the communication control unit (telephone) 52 and 53, the data management unit 54, the input/output unit 55, the charging process unit 56 and the like.

The data measuring unit 51 performs a measurement of a subject's health based on body fluid and the like. Here, as shown in FIG. 1(b), the data measuring unit 51 includes the sensor 510 and the calculation control unit 511. The calculation control unit 511 converts data to data added with a unit concerning health (described as "health care data" below) based on said data being numerical data acquired by the sensor 510 and calibration curve data held by the calculation control unit 511. Here, the "numerical data" means data indicating the electric quantity (for example, a voltage value, a current value, a resistance value or a power value) outputted by the sensor 510, and it is not clear which characteristic of health is indicated by referring to only said "numerical data". Also, the "calibration curve data" means the data showing a straight line or a curved line for describing the relation between the "numerical data" and the characteristics of the health which is a measuring subject.

The communication control units 52 and 53 control the communication between the data measuring unit 51 and the data management unit 54. The data management unit 54 accumulates and manages the health care data received from the data measuring unit 51 via the communication control units 52 and 53, and controls the input/output unit 55 and the charging process unit 56.

The medical information service system 500 comprising such structure as described above employs a method in which the data measuring unit 51 acquires health care data, for example, such as "mg Hg" as blood pressure level and "mg/dl" as blood glucose level, and transmits the health care data, via a telephone line and the like, to the data management unit 54 installed in a medical institution and the like. A doctor, a nurse and the like (described as "medical representatives" below) checks and manages the health condition by referring to changes of the health care data from the past to the present which is accumulated in the data management unit 54 via the input/output unit 55. Also, when a medical representative provides a diagnosis or advice based on the health care data to a subject via the input/output unit 55, the architect and the like of the present system 500 can make profits by charging for the diagnosis or advice and the transmission of the health care data from the data measuring unit 51 to the data management unit 54 through the charging process unit 56.

The outline of the medical information service system 500 using the health care data according to the conventional technology is described above. As shown in FIG. 1(b), the data measuring unit 51 of the medical information service system 500 includes the sensor 510 which directly measures a subject and the calculation control unit 511 (for example, a micro computer which includes ROM holding a calculation algorithm or RAM) which converts the numerical data acquired from the sensor 510 into health care data.

In other words, the data measuring unit 51 needs an exclusive IC and the like for converting the numerical data detected by the sensor 510 into health care data. Thus, there is a problem that the cost of the data measuring unit 51 increases.

In addition, as the communication control unit 52, by using a cellular phone which has been noticeably distributed in recent years, the transmission of the health care data is made even easier. However, because the data measuring unit 51 needs to include the calculation control unit 511, there is a problem that the size of the data measuring unit 51 becomes large. Such large size contradicts portability of a cellular phone, and it becomes an obstacle when a user attempts to acquire health care data easily at any time and anywhere.

Also, if the version of the sensor 510 included in the data measuring unit 51, for example, such as a blood glucose measuring sensor changes, it causes necessity to replace the whole data measuring unit 51. This increases a user's burden.

Moreover, in the future, in the case where it is necessary to provide "point of care (POC)" (for example, an urgent blood component analysis in the medical spot and the like), even if the sensor 510 of the data measuring unit 51 is transformed to a chip and made small and light, and an advanced algorithm is necessary for health care data analysis, a computer capable of a high external processing has to be employed. In such case as described above, a fast operation is not possible, and a necessary analysis and the like can not be easily performed at any time and anywhere.

The object of the present invention, in view of the above problem, is to provide a data measuring apparatus, a health care acquisition system and the like which are capable of reducing the cost of the measuring apparatus for acquiring health care data and a simple and easy acquisition and processing of health care data.

### Disclosure of Invention

In order to achieve the above mentioned object, the data measuring apparatus according to the present invention measures a predetermined biological characteristic of a subject. And, the data measuring apparatus includes: a characteristic conversion unit operable to convert a characteristic of a subject into information indicating predetermined electric quantity; an information transmission unit operable to transmit the information indicating the converted electric quantity to another predetermined apparatus; and a data reception unit operable to receive data which is added with a unit indicating the biological characteristic of the subject from said another apparatus.

Thus, the data measuring apparatus according to the present invention is formed so that only the numerical data which is the original data of the health care data of a subject is detected and transmitted to an external apparatus and the external apparatus receives the calculated health care data. Consequently, the data measuring apparatus can be simplified and the cost can be reduced.

Also, in order to achieve the above mentioned object, the health care data acquisition system according to the present invention comprises: a data measuring apparatus for measuring a biological characteristic of a subject and a data management apparatus for managing data of said characteristic, said data measuring apparatus and data management apparatus being connected to each other via a network, wherein said data measuring apparatus includes: a characteristic conversion unit operable to convert a characteristic of a subject into information indicating predetermined electric quantity; and an information transmission unit operable to transmit the information indicating the electric quantity to said data management apparatus, and said data management apparatus includes: a reception unit operable to receive the information from said data measuring apparatus; a data conversion unit operable to convert said electric quantity into data which is added with a unit indicating the biological characteristic of the subject, based on said received information; and a return unit operable to return said converted data to said data measuring apparatus.

Thus, the health care data acquisition system is formed so that only the numerical data which is the original data of the health care data is detected by the data measuring apparatus which is used by a subject and the calculation of the health care data is performed by the external data management apparatus. Consequently, the data measuring apparatus can be simplified and the cost can be reduced. Furthermore, the calculation of the health care data can be flexibly performed by the side of the external data management apparatus.

In addition, in order to achieve the above mentioned object, the present invention can be realized as a data measuring method comprising steps of the characteristic units of the data measuring apparatus or as a program comprising all the steps of the method. And, the program cannot only be stored in ROM and the like which are included in the apparatus capable of realizing the above mentioned method, but also the program can be distributed via a record medium such as a CD-ROM and the like and a transmission medium such as a communication network and the like. Also, in order to achieve the above mentioned object, the present invention can be realized as a health care data acquisition method comprising steps of the characteristic units of the health care data acquisition system.

### Further Information about Technical Background to this Application

The disclosure of Japanese Patent Application No. 2002-233369 filed on August 9, 2002 including specification, drawings and claims is incorporated herein by reference in its entirety.

### Brief Description of Drawings

These and other objects, advantages and features of the invention will become apparent from the following description thereof taken in conjunction with the accompanying drawings that illustrate a specific embodiment of the invention. In the Drawings:
FIG. 1 is a block diagram showing a functional structure of the conventional medical information service system;
FIG. 2 is a block diagram showing a functional structure of the health care data acquisition system according to the first embodiment;
FIG. 3 is a diagram showing an example of the calibration curve data;
FIG. 4 is an example of a structure of data exchanged between the data measuring apparatus and the data management apparatus;
FIG. 5 is a communication sequence diagram according to the first embodiment;
FIG. 6 is a communication sequence diagram according to the variation example of the first embodiment;
FIG. 7 is a block diagram showing a functional structure of the health care data acquisition system according to the second embodiment; and
FIG. 8 is a communication sequence diagram according to the second embodiment.

### Best Mode for Carrying Out the Invention

The embodiments of the present invention will be explained by referring to the drawings as following.

### (First Embodiment)

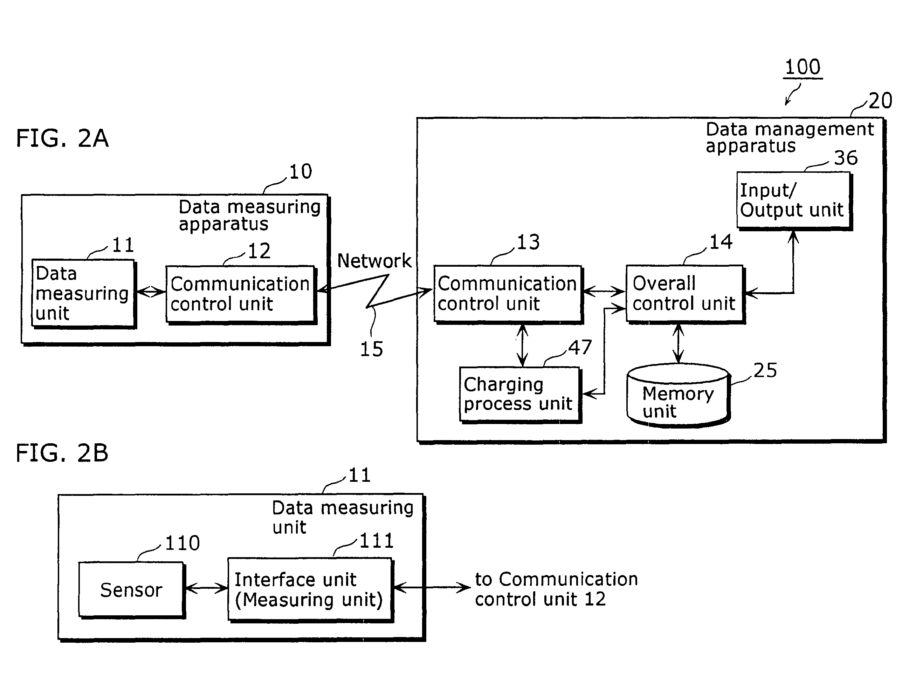

FIG. 2(a) is a block diagram showing a functional structure of the health care data acquisition system 100 according to the first embodiment. As shown in FIG. 2(a), the health care data acquisition system 100 comprises the data measuring apparatus 10 and the data management apparatus 20.

First, the data measuring apparatus 10 will be explained.

The data measuring apparatus 10 includes the data measuring unit 11 and the communication control unit 12. FIG. 2(b) is a block diagram showing a functional structure of the data measuring unit 11. As shown in FIG. 2(b), the data measuring unit 11 includes the sensor 110 and the interface unit (measuring unit) 111. The sensor 110 measures predetermined characteristics (for example, the blood glucose level in blood, the protein content in urine and the like) of the body fluid and the like of a subject. Then, the sensor 110 converts the characteristics into electrical quantity (for example, a voltage value, a current value, a resistance value, a power value and the like). And, the sensor 110 acquires numerical data indicating the electrical quantity.

The interface unit 111 transmits the numerical data acquired by the sensor 110 to the communication control unit 12 according to the predetermined order.

The communication control unit 12 is, for example, a cellular phone, and includes a micro computer which has ROM, RAM and the like. And, the communication control unit 12 controls the communication with the data management apparatus 20 which is connected to the communication control unit 12 via the network 15 such as a telephone line and the like. In addition, the communication control unit 12 directs the data measuring unit 11 to acquire the numerical data. Moreover, the communication control unit 12 receives the numerical data from the data measuring unit 11, and transmits it to the data management apparatus 20. And, the communication control unit 12 receives the health care data from the data management apparatus 20.

Next, the data management apparatus 20 will be explained.

The data management apparatus 20 is, for example, a personal computer, and includes the communication control unit 13, the overall control unit 14, the memory unit 25, the input/output unit 36 and the charging process unit 47.

The communication control unit 13 is a large-scale integration (LSI) for communication control, including, for example, ROM, RAM, and the like, and controls the communication between the data measuring apparatus 10 and the data management apparatus 20. Also, in the case where there is data transmission from the data measuring apparatus 10 to the data management apparatus 20 or data transmission from the data management apparatus 20 to the data measuring apparatus 10, the communication control unit 13 notifies the charging process unit 47 of the information concerning such data transmissions as described above (for example, the time spent for the data transmission, a telephone number and the like).

The overall control unit 14 is, for example, a micro computer including ROM, RAM, and the like, and controls the overall data management apparatus 20. And, the overall control unit 14 converts the numerical data received from the data measuring apparatus 10 into health care data via the communication control unit 13. More specifically, the overall control unit 14 converts the numerical data into health care data by using the calibration curve.

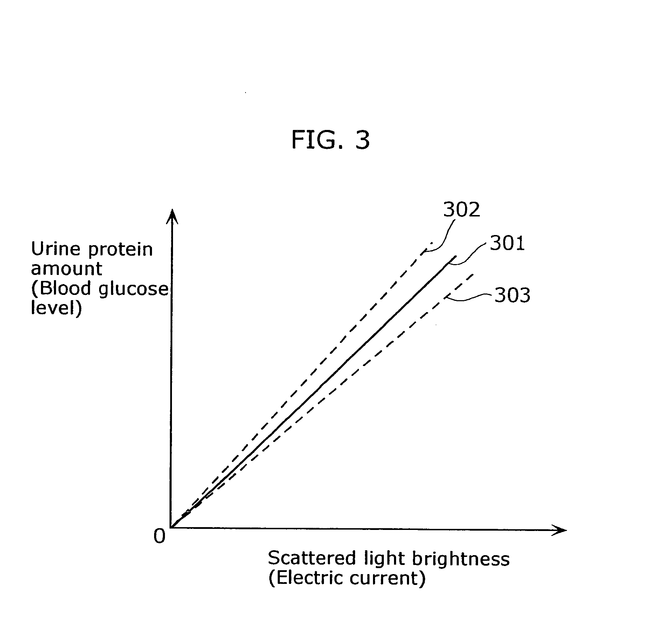

FIG. 3 is a diagram showing an example of the calibration curve data which is used by the overall control unit 14 when converting the numerical data into the health care data. As shown in FIG. 3, the health care data for the numerical data is univocally defined. a

The memory unit 25 memorizes respective kind of calibration curve data by corresponding the data to a version or a lot of the sensor 110 or the measuring unit 111. In addition, the memory unit 25 accumulates, by corresponding to a subject, the numerical data received from the data measuring apparatus 10 and the health care data converted by the overall control unit 14. Thus, the overall control unit 14 can convert chronological changes of the accumulated health care data into diagrams and graphs, and provide them to the input/output unit 36. And, the overall control unit 14 can return such diagrams and graphs as described above to the data measuring apparatus 10. Therefore, the subject who is a user of the data measuring apparatus 10 can receive the health care data from the data management apparatus 20 and check the health care data at any time via a display included in the communication control unit 12 (for example, a cellular phone).

The charging process unit 47 manages the communication state of the present system 100 and sets the charge for the user of the data measuring apparatus 10 or the medical representative of the data management apparatus 20, based on the number of accesses from the data measuring apparatus 10 to the data management apparatus 20, the number of operations of the input/output unit 36 by the medical representative and the like. The result of the charge is reported to the user or the medical representative every time the data is communicated or every predetermined period. More specifically, the charging process unit 47 is connected to the communication control unit 13 and the overall control unit 14, and detects the data transmission from the data measuring apparatus 10 to the data management apparatus 20 and the data transmission from the data management apparatus 20 to the data measuring apparatus 10 (for example, the information about the data transmissions (such as a telephone number, the time spent for the data transmissions and the like) provided by the communication control unit 13). And, the charging process unit 47 calculates the time spent for the data transmissions and the number of the data transmissions. Moreover, the charging process unit 47 performs a charging process for at least either one or both of the data measuring apparatus 10 or the data management apparatus 20.

Therefore, by installing the charging process unit 47, when providing the health care data acquisition system 100, the compensation for the provided service can be acquired from both the user who uses the data measuring apparatus 10 and the medical representative who uses the data management apparatus 20.

The input/output unit 36 is, for example, a key board or a liquid crystal display (LCD) apparatus, and transmits the information indicating the operations received from the medical representative to the overall control unit 14. And, the input/output unit 36 displays the information indicating the communication state received via the overall control unit 14, respective kind of data and the like. More specifically, the input/output unit 36 converts the chronological changes of the accumulated health care data into diagrams and graphs, and provides them to the medical representative. Also, the input/output unit 36 receives data input for a diagnosis or advice from the medical representative. The subject who is a user of the data measuring apparatus 10 can check such data as described above by the display and the like included in the communication control unit 12 (for example, a cellular phone).

More importantly, the numerical data received from the data measuring apparatus 10 cannot be modified by the user. Thus, the medical representative can remove the operation such as an arbitrary self-assessment (for example, "I neglected my health last night, so by changing data a little..." and so on) from the data measuring apparatus 10. Then, the medical representative can correctly acquire the health care data, and provide an accurate diagnosis or an appropriate advice. This is because the numerical data acquired by the data measuring unit 11 is automatically transmitted to the data management apparatus 20 via the communication control unit 12.

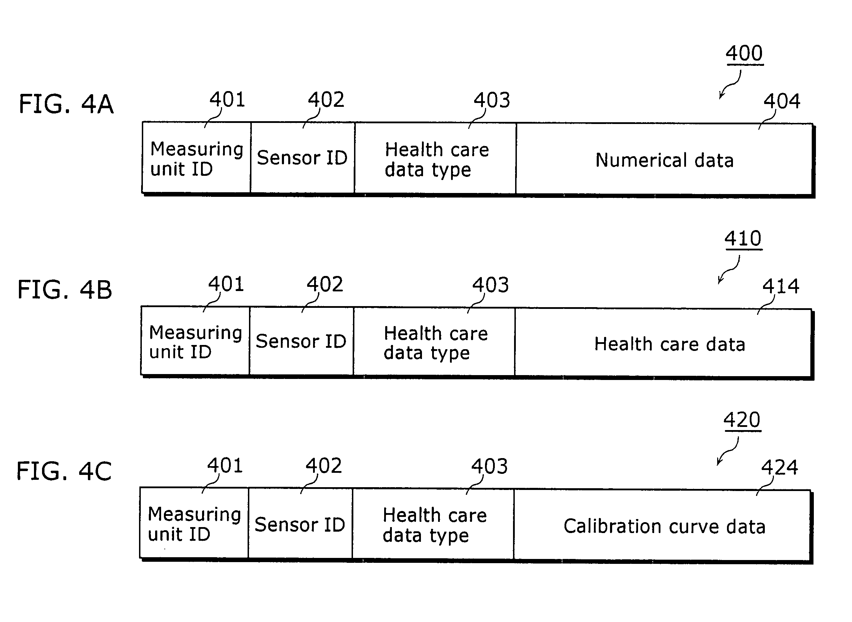

FIG. 4(a)-(c) are diagrams showing structure examples of the data exchanged between the data measuring apparatus 10 and the data management apparatus 20. FIG. 4(a) is a diagram showing a structure example of the data 400 including the numerical data 404 which is transmitted from the data measuring apparatus 10 to the data management apparatus 20. The data 400 includes the measuring unit ID 401, the sensor ID 402 and the health care data type 403 in addition to the numerical data 404.

FIG. 4(b) is a diagram showing a structure example of the data 410 including the health care data 414 which is transmitted from the data management apparatus 20 to the data measuring apparatus 10. As well as such data 400 as described above, the data 410 includes the measuring unit ID 401, the sensor ID 402 and the health care data type 403.

Also, FIG. 4(c) is data used in the later mentioned variation according to the first embodiment, and a diagram showing a structure example of the data 420 including the calibration curve data which is transmitted from the data management apparatus 20 to the data measuring apparatus 10. As well as the above mentioned data 400, the data 420 includes the measuring unit ID 401, the sensor ID 402 and the health care data type 403.

Next, the operations performed by the health care data acquisition system 100 which is formed as described above will be explained.

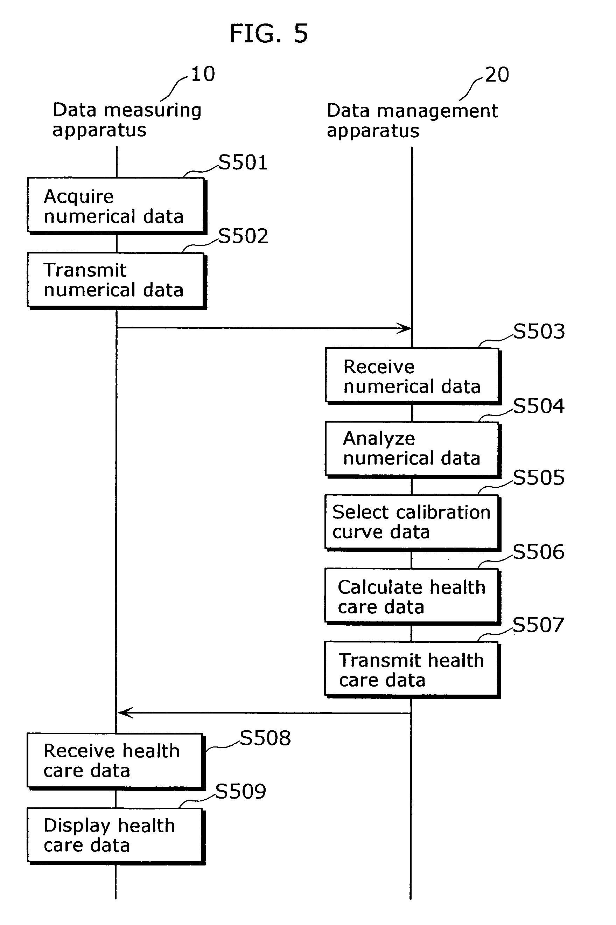

FIG. 5 is a communication sequence diagram in the case where the health care data converted from the numerical data by using the calibration curve data is transmitted from the data management apparatus 20 to the data measuring apparatus 10.

First, the data measuring unit 11 of the data measuring apparatus 10 measures the blood or urine of a subject in the predetermined procedures, and acquires the numerical data (S501). Specifically, when the sensor 110 of the data measuring unit 11 receives a direction from the communication control unit 12 via the interface unit 111, the numerical data which indicates the electric quantity such as the current value, voltage value, resistance value power value, and the like is outputted to the communication control unit 12 via the interface unit 111. For example, in the case where the blood pressure is measured, the sensor 110 outputs the numerical data of the voltage value as the measurement result, according to the elements such as a piezoelectric element, a thermocouple and the like. Also, the communication control unit 12 of the data measuring apparatus 10 transmits the numerical data acquired by the data measuring unit 11 to the data management apparatus 20 (S502).

Thus, when the overall control unit 14 of the data management apparatus 20 receives the numerical data from the data measuring apparatus 10 via the communication control unit 13 (S503), the overall control unit 14 analyzes the content of the received numerical data (S504). Moreover, the overall control unit 14 of the data management apparatus 20 selects the calibration curve data based on the above mentioned analysis result (S505), and converts the numerical data into the health care data by using the calibration curve data (S506). More specifically, when the overall control unit 14 receives the numerical data, it converts the numerical data into the health care data by using the calibration curve data which is selected based on the predetermined analysis algorithm. For example, in the case where the blood pressure is measured, the overall control unit 14 selects the calibration curve data indicating the already known blood pressure level which corresponds to the received numerical data, and converts the numerical data into the health care data by using the calibration curve data. After that, the overall control unit 14 of the data management apparatus 20 directs the communication control unit 13 to transmit the health care data to the data measuring apparatus 10 (S507).

After that, when the data measuring apparatus 10 receives the health care data from the data management apparatus 20 (S508), the data measuring apparatus 10 displays the health care data on the display (a drawing is omitted here) of the communication control unit 12 (S509).

Thus, under the health care data acquisition system 100 according to the first embodiment, the data measuring apparatus 10 detects only the numerical data which is the original data for calculating the health care data of a subject. And, the calculation of the health care data is performed by the external data management apparatus 20. Thereby, the data measuring apparatus 10 can be simplified and the cost can be reduced. In other words, in the case where the health care data acquisition system is realized, said system comprising the communication control unit 12 (for example, a cellular phone) which is combined with the data measuring unit 11 (for example, a blood glucose level measuring unit) and the data management apparatus 20 (for example, a personal computer) which includes a health care data producing function, a user who uses the communication control unit 12 (for example, a cellular phone) can perform an advanced analysis by easily using the very simple apparatus.

Although the embodiment example in which a blood pressure level is acquired as health care data is explained according to the above mentioned embodiment, the case in which a blood glucose level is acquired as health care data will be explained as following. In such case as described above, the sensor 110 can be a disposable sensor which includes a reagent for detecting the numerical data, and the overall control unit 14 can include a reading apparatus. In this case, the numerical data acquired by the sensor 110 and the calibration curve data memorized in the overall control unit 14 can be used to calculate the health care data.

Moreover, the sensor 110 is explained as including a reagent. However, if the reagent is improved, the version of the sensor 110 changes. Thus, it is necessary that the calibration curve data within the overall control unit 14 be adjusted to the new sensor.

According to the first embodiment, in order to cope with above mentioned problem, the version number or the lot number is set for the sensor 110 or the measuring unit 111. And, the calibration curve data according to the version number or the lot number of the sensor (these are called "attribute information" or "property information") is stored in the overall control unit 14.

In such case as described above, the data measuring unit 11 transmits the version number or the lot number of the sensor 110 or the measuring unit 111, as well as the detected numerical data, to the overall control unit 14. The overall control unit 14 of the data management apparatus 20 which has received the attribute information from the data measuring apparatus 10 refers to the acquired version number or lot number, and selects the corresponding calibration curve data. And, the overall control unit 14 calculates health care data. And, the overall control unit 14 transmits the calculated health care data to the data measuring apparatus 10.

In other words, in addition to improving the performance of the data measuring apparatus 10 (that is, the sensor or the measuring unit), the improvement of the measuring accuracy can be attempted by catching up on the side of the overall control unit 14. Thus, the cost can be prevented from influencing the data measuring unit 11, thereby it is possible to build a very inexpensive system for a user.

### (Variation)

Although the case where the health care data is calculated on the side of the data management apparatus 20 is explained in the above mentioned embodiment example according to the first embodiment, there might be a case where the data measuring apparatus 10 acquires the calibration curve data from the data management apparatus 20 based on the attribute information such as the sensor and the like and calculates the health care data on the side of the data measuring apparatus 10.

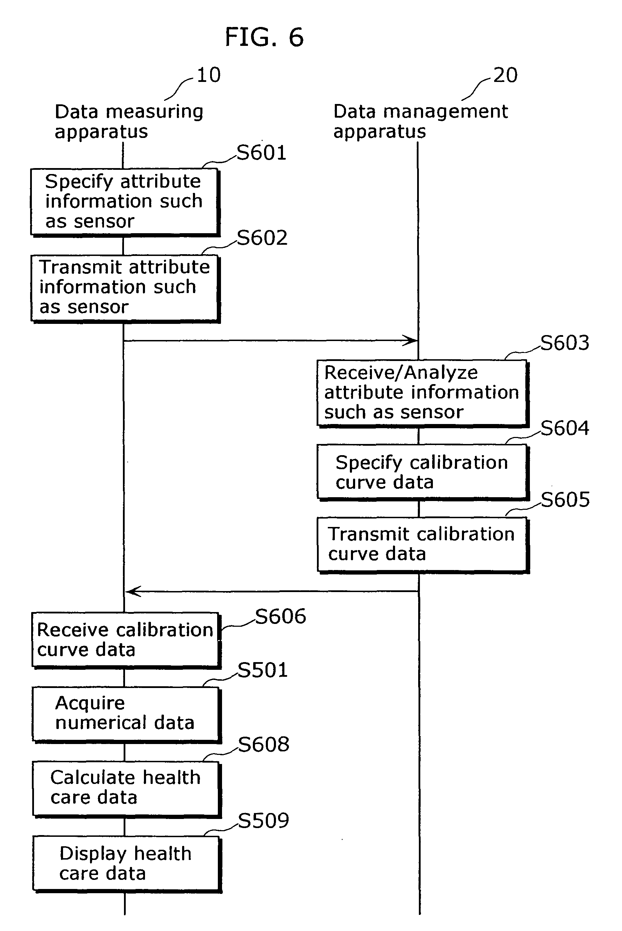

FIG. 6 is a communication sequence diagram in the case where the data measuring apparatus 10 acquires the calibration curve data from the data management apparatus 20 based on the attribute information such as the sensor and the like, and calculates the health care data in the data measuring apparatus 10.

First, the data measuring apparatus 10 specifies the attribute information (for example, the version number or the lot number) of the sensor 110 or the measuring unit 111 according to the direction of the communication control unit 12 (S601). Also, the data measuring apparatus 10 transmits the specified attribute information to the data management apparatus 20 (S602).

Thus, the overall control unit 14 of the data management apparatus 20 receives the attribute information from the data measuring apparatus 10 via the communication control unit 13 (S603), and specifies the calibration curve data based on the received attribute information (S604). Moreover, the data management apparatus 20 transmits the specified calibration curve data to the data measuring apparatus 10 (S605).

After that, the data measuring apparatus 10 which has transmitted the calibration curve data (S606) acquires the numerical data via the data measuring unit 11 (S501), and produces the health care data based on the acquired numerical data and the received calibration curve data (S502). And, the data measuring apparatus 10 displays the health care data on the display (drawing omitted) of the communication control unit 12 (S509).

As described above, according to the health care data acquisition system of the first embodiment, the data measuring apparatus 10 acquires the necessary calibration curve data based on the sensor and attribute information of the measuring unit from the data management apparatus each time. And, the memory for accumulating much calibration curve data corresponding to the version number or the lot number is unnecessary. Thereby, it is possible to realize the data measuring apparatus with a small size.

In addition, according to the health care data acquisition system 100 of the first embodiment, a user who is a subject can immediately acquire the health care data by using the simple data measuring apparatus combined with a cellular phone and the like. And, the user can easily compare the past data and the present data by receiving a series of health care data from the data management apparatus.

According to the first embodiment as described above, the charging process unit 47, after setting the charge, reports it to the data measuring apparatus 10 or the overall control unit 14, via the communication control unit 13. However, the report can be made not only by a communication line, but also by mail and the like.

Furthermore, although the first embodiment is explained as that the communication through a cellular phone is performed by a cellular phone line, the communication line is not limited to this. For example, an e-mail and the like can be used through the Internet. Consequently, the cost for communication can be reduced, and the connection fee can be made inexpensive.

### (Second Embodiment)

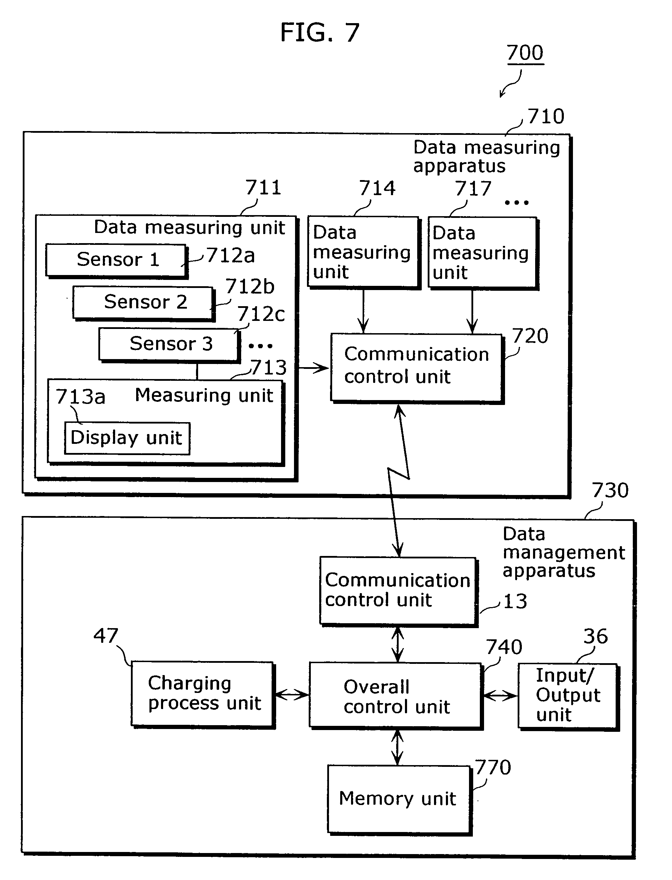

FIG. 7 is a block diagram showing a functional structure of the health care data acquisition system 700 according to the second embodiment. The present system 700 is different from the health care data acquisition system 100 according to the first embodiment in that the data measuring apparatus comprises a plurality of data measuring units and a plurality of sensors.

The operations performed by the present system 700 are basically the same as those of the health care data acquisition system 100 according to the first embodiment. The present system 700 converts the numerical data measured by the data measuring units 711, 714 and 717 into the health care data in the overall control unit 740, and memorizes it in the memory unit 770. Furthermore, a medical representative inputs data such as a diagnosis and advice for the health care data and the chronological changes thereof via the input/output unit 36. And, a user of the data measuring apparatus 710 checks the above mentioned data.

Also, according to the second embodiment, as a use method of the health care data acquisition system 700, the charging process unit 47 monitors the communication state, and sets the charge for the user of the data measuring apparatus 710 and the medical representative, based on the number of accesses from the data measuring apparatus 710 to the overall control unit 740, the number of operations of the input/output unit 36 by the medical representative and the like. The result of the charge can be reported every time the data is transmitted, or all together every predetermined period, to the user's terminal, that is, the data measuring apparatus 710 or the overall control unit 740.

In addition, as the second embodiment uses a plurality of sensors and a plurality of measuring units, based on the ID number which distinguishes the sensors and measuring units, which sensor is set for which measuring unit is distinguished by the side of the overview control unit 740. After distinguishing, the corresponding calibration curve data is selected from the memory unit 770, and the numerical data received from the data measuring apparatus 710 is converted into the health care data.

As described above, according to the second embodiment, by enabling the analysis of multiple health care data, even higher value-added information can be distributed.

Next, the operations performed by the health care data acquisition system 700 formed as described above will be explained.

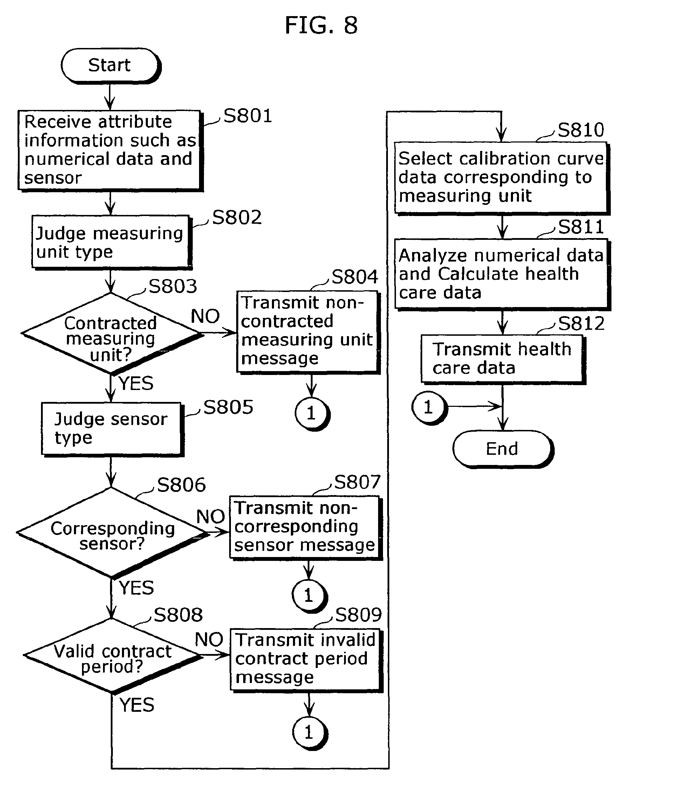

FIG. 8 is a flow chart showing a flow of the procedures performed by the data management apparatus 730 of the health care data acquisition system 700.

First, the overall control unit 740 receives the numerical data via the communication control unit 13 (S801), and judges the type of the measuring unit (S802). Here, the overall control unit 740 judges whether or not the current measuring unit is the one which the measuring unit 713 made a contract with (S803). In the case where the current measuring unit is not the one the measuring unit 713 made a contract with (S803: NO), the overall control unit 740 transmits a message indicating the fact to the data measuring apparatus 710 (S804).

In the case where the current measuring unit is the one which the measuring unit 713 made a contract with (S804: YES), the overall control unit 740 judges the type of the sensor (S805), and judges whether or not the sensor corresponds to the measuring unit (S806). Thus, in the case where the sensor does not correspond to the measuring unit (S806: NO), the overall control unit 740 transmits a message indicating the fact to the data measuring apparatus 710 (S807).

Moreover, the overall control unit 740 judges whether or not the current measuring unit has a valid contract period (S808). In the case where the current measuring unit does not have a valid contract period (S808: NO), the overall control unit transmits a message indicating the fact to the data measuring apparatus 710 (S809).

Finally, the overall control unit 740 selects the calibration curve data corresponding to the above mentioned measuring unit (S810), and produces health care data based on the received numerical data and the calibration curve data (S811). And, the overall control unit 740 transmits the health care data to the data measuring apparatus 710 (S812).

As described above, according to the present invention, if a user connects a measuring unit including a very simple structure to a cellular phone, health care data can be exchanged as a health care system. And, the image of the conventional individual health care apparatus can be greatly changed. Then, a solid and simple health care system including service for a user can be provided.

According to each embodiment as described above, the data measuring units 11, 711, 714 and 717 are examples of the characteristic conversion unit of the present invention. The sensors 110, 712a, 712b and 712c are examples of the sensor included in the conversion unit according to the present invention. The communication control units 12 and 720 of the data measuring apparatus 10 and 710 are examples of the information transmission unit and the data reception unit of the data measuring apparatus according to the present invention. Also, the communication control unit 13 of the data management apparatuses 20 and 730 is an example of the reception unit and the return unit of the data management apparatus according to the present invention. The overall control units 14 and 740 are examples of the data conversion unit according to the present invention. The memory units 25 and 770 are examples of the memory unit according to the present invention. The input/output unit 36 is an example of the direction reception unit according to the present invention. The charging process unit 47 is an example of the charging unit according to the present invention. In addition, the communication control unit 13 and the charging process unit 47 of the data management apparatuses 20 and 730 are examples of the first detection unit according to the present invention. The communication control unit 13 and the charging process unit 47 of the data management apparatuses 20 and 730 are examples of the second detection unit according to the present invention. Moreover, the numerical data corresponds to the information indicating the predetermined electric quantity which is converted by the characteristic conversion unit according to the present invention. The health care data corresponds to the data which is added with the unit indicating the whole or partial predetermined characteristics of medicine, physiology and biology according to the present invention. The version number corresponds with the property information according to the present invention.

Furthermore, the present invention is not limited to the above mentioned embodiments. The data measuring apparatus according to the present invention can be realized by using a personal digital assistant (PDA) other than a cellular phone. Also, the unit for display and reception can be omitted, and a transmission only terminal which includes only the information transmission unit according to the present invention can be used. In this case, the health care data can be only checked by the overall control unit 14.

As an example of the health care data, according to the above mentioned embodiments, blood pressure and a blood glucose level are raised. However, other examples such as a body temperature, heart rate, blood pressure and the like can be used.

Also, the living organisms according to the present invention include livestock, animals such as pets, agricultural crops and plants such as house plants and the like other than humans.

The property information according to the present invention can be the information other than the version number or the lot number, as long as it can distinguish the performance and features of the sensor.

In addition, the present invention is a program for implementing the function of the whole or partial unit (or an apparatus, an element, a circuit and the like) of the above mentioned health care data acquisition system according to the present invention by a computer, and it can be a program which operates in cooperation with a computer.

Moreover, the present invention is a medium that supports a program for implementing the whole or partial function of the whole or partial unit of the above mentioned health care data acquisition system according to the present invention by a computer. And, the present invention can be a medium that supports the program which can be read by a computer and has been read by a computer and implements the function in cooperation with the computer.

Also, the partial unit (or an apparatus, an element, a circuit, a unit and the like) according to the present invention and the partial step (or a process, an operation, an action and the like) means several units or steps of a plurality of the units or steps, or a partial function or a partial operation of one unit or step.

In addition, a partial apparatus (or an element, a circuit, a unit and the like) according to the present invention means several apparatuses of a plurality of the apparatuses, a partial unit (or an element, a circuit, a unit and the like) of one apparatus, or a partial function of one unit.

The present invention also includes a record medium which holds the recorded program according to the present invention and can be read by a computer.

The program according to the present invention can be recorded in the record medium which can be read by a computer, and operate in cooperation with the computer.

Also, the program according to the present invention can be transmitted in the transmission medium, and can be read by a computer. And, the program can operate in cooperation with the computer.

A data structure of the present invention includes: a data base, a data format, a data table, a data list, a data type and the like.

In addition, a record medium includes ROM and the like. A transmission medium includes a transmission device such as the Internet and the like, a light, an electromagnetic wave, an acoustic wave and the like.

The above-mentioned computer according to the present invention may include a firmware, an operating system (OS) and a peripheral device.

As described above, the structure according to the present invention can be realized both as soft ware and as hard ware.

Although only some exemplary embodiments of this invention have been described in detail above, those skilled in the art will readily appreciate that many modifications are possible in the exemplary embodiments without materially departing from the novel teachings and advantages of this invention. Accordingly, all such modifications are intended to be included within the scope of this invention.

### Industrial Applicability

The present invention is beneficial for a portable data acquisition system and the like for measuring characteristics of health or biology. In particular, the present invention can be applied to a system and the like under which a measuring unit or a sensor needs to change calibration curve data by a manufacturing version or a manufacturing lot.

## Claims

1. A data measuring apparatus for measuring a predetermined biological characteristic of a subject, the apparatus including:
a characteristic conversion unit operable to convert a characteristic of a subject into information indicating predetermined electric quantity;
an information transmission unit operable to transmit the information indicating the converted electric quantity to another predetermined apparatus; and
a data reception unit operable to receive, from said another apparatus, data which is added with a unit indicating the biological characteristic of the subject.

2. A health care data acquisition system comprising a data measuring apparatus for measuring a biological characteristic of a subject and a data management apparatus for managing data of said characteristic, said data measuring apparatus and data management apparatus being connected to each other via a network,
wherein said data measuring apparatus includes:
a characteristic conversion unit operable to convert a characteristic of a subject into information indicating predetermined electric quantity; and
an information transmission unit operable to transmit the information indicating the electric quantity to said data management apparatus, and
said data management apparatus includes:
a reception unit operable to receive the information from said data measuring apparatus;
a data conversion unit operable to convert said electric quantity into data which is added with a unit indicating the biological characteristic of the subject, based on said received information; and
a return unit operable to return said converted data to said data measuring apparatus.

3. The health care data acquisition system according to Claim 2,
wherein said characteristic conversion unit has:
a conversion unit including a sensor which converts said characteristic into a predetermined electric quantity; and
an interface unit including a measuring unit for producing information which indicates said converted electric quantity,
wherein said sensor and said measuring unit are respectively corresponded with property information indicating respective attribute,
said characteristic conversion unit further specifies said property information,
said information transmission unit further transmits said predetermined property information to said data management apparatus, and
said data conversion unit of said data management apparatus converts said property information into health care data taking said property information into account.

4. The health care data acquisition system according to Claim 3,
wherein said data conversion unit of said data management apparatus performs said conversion based on calibration curve data which relates said predetermined electric quantity to said characteristic of the subject.

5. The health care data acquisition system according to Claim 4,
wherein said data management apparatus further includes a memory unit operable to memorize calibration curve data corresponding to said property information, and
said data conversion unit selects calibration curve data from said memory unit, according to said property information, and performs said conversion based on the selected calibration curve data.

6. The health care data acquisition system according to Claim 5,
wherein said memory unit memorizes calibration curve data which corresponds to a pair of said property information of the sensor and said property information of the measuring unit.

7. The health care data acquisition system according to Claim 6,
wherein said data management apparatus further includes a direction reception unit operable to receive a direction from an operator, and
said memory unit further memorizes new calibration curve data which corresponds to a pair of new property information of said sensor and new property information of said measuring unit, based on said direction.

8. The health care data acquisition system according to Claim 7,
wherein said property information of the sensor is an ID number capable of specifying a current sensor, and
said property information of the measuring unit is an ID number capable of specifying a current unit.

9. The health care data acquisition system according to Claim 7,
wherein said property information of the sensor indicates at least one of the following information: a type of body fluid which is a measuring subject of the current sensor, a manufacturing number or a manufacturing lot number of a measuring unit which matches the current sensor, and a valid period of the current sensor.

10. The health care data acquisition system according to Claim 7,
wherein said property information of the measuring unit indicates at least one of the following information: a type of body fluid which is a measuring subject of the current measuring unit, a manufacturing number or a manufacturing lot number of a sensor which matches the current measuring unit, and a valid period of the current measuring unit.

11. The health care data acquisition system according to Claim 2,
wherein said data management apparatus further includes:
a first detection unit operable to detect that said data measuring apparatus has transmitted the information to said data management apparatus;
a second detection unit operable to detect that said data management apparatus has transmitted the health care data to said data measuring apparatus; and
a charging unit operable to charge a predetermined amount to at least one of said data measuring apparatus and said data management apparatus in the case where each of said transmissions is detected by said first detection unit and said second detection unit.

12. The health care data acquisition system according to Claim 11,
wherein said first detection unit further calculates the number of times the information has been transmitted,
said second detection unit further calculates the number of times the health care data has been transmitted, and
said charging unit charges the predetermined amount according to respective number of times the information has been transmitted as calculated by the first detection unit and the health care data has been transmitted as calculated by the second detection unit.

13. A data measuring method for measuring a predetermined biological characteristic of a subject, the method comprising:
a characteristic conversion step for converting a characteristic of a subject into information indicating predetermined electric quantity;
an information transmission step for transmitting the information indicating the converted electric quantity to another predetermined apparatus; and
a data reception step for receiving data which is added with a unit indicating the biological characteristic of the subject from said another apparatus.

14. A health care data acquisition method for a system comprising a data measuring apparatus for measuring a biological characteristic of a subject and a data management apparatus for managing data of said characteristic, said data measuring apparatus and data management apparatus being connected to each other via a network,
wherein in said data measuring apparatus the following steps are performed:
a characteristic conversion step for converting a characteristic of a subject into information indicating predetermined electric quantity; and
an information transmission step for transmitting the information indicating the electric quantity to said data management apparatus, and
in said data management apparatus the following steps are performed:
a reception step for receiving the information from said data measuring apparatus;
a data conversion step for converting said electric quantity into data which is added with a unit indicating the biological characteristic of the subject, based on said received information; and
a return step for returning said converted data to said data measuring apparatus.

15. A program for a data measuring apparatus which measures a predetermined biological characteristic of a subject, the program comprising:
a characteristic conversion step for converting a characteristic of a subject into information indicating predetermined electric quantity;
an information transmission step for transmitting the information indicating the converted electric quantity to another predetermined apparatus; and
a data reception step for receiving data which is added with a unit indicating the biological characteristic of the subject from said another apparatus.
